(19) Europäisches Patentamt
European Patent Office
Office européen des brevets

(11) Publication number: **0 188 043**
**B1**

(12) # EUROPEAN PATENT SPECIFICATION

(45) Date of publication of patent specification: **21.12.88**

(51) Int. Cl.[4]: **C 07 C 45/53**, C 07 C 179/02, C 07 C 29/132

(21) Application number: **85300265.7**

(22) Date of filing: **15.01.85**

(54) **Improvements in the catalysis of the oxidation of hydrocarbons to form hydroperoxides and/or the decompostion of the hydroperoxides.**

(43) Date of publication of application:
**23.07.86 Bulletin 86/30**

(45) Publication of the grant of the patent:
**21.12.88 Bulletin 88/51**

(84) Designated Contracting States:
**BE DE FR GB IT NL**

(56) References cited:
**FR-A-2 267 299**
**GB-A- 988 006**
**GB-A-1 191 573**
**US-A-3 927 105**

**CHEMICAL ABSTRACTS, vol. 94, no. 19, 2nd May 1981, page 622, no. 156544g, Columbus, Ohio, US; & JP - A - 80 151 553 (MITSUI PETROCHEMICAL INDUSTRIES LTD.) 26-11-1980**

(73) Proprietor: **ATLANTIC RICHFIELD COMPANY**
**515 South Flower Street**
**Los Angeles California 90071 (US)**

(72) Inventor: **Taylor, Paul D.**
**1128 Cymry Drive**
**Berwyn Pennsylvania 19312 (US)**
Inventor: **Mocella, Michael T.**
**308 Baywood Road**
**West Chester Pennsylvania 19380 (US)**

(74) Representative: **Cropp, John Anthony David et al**
**MATHYS & SQUIRE 10 Fleet Street**
**London, EC4Y 1AY (GB)**

**EP 0 188 043 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention is concerned generally with the production of oxidation products, especially alcohols and/or ketones, by oxidation of a hydrocarbon in the presence of a molecular oxygen containing gas to produce a reaction mixture containing the corresponding hydroperoxide and decomposition of the resultant hydroperoxide.

In accordance with the invention either or both of the oxidation and decomposition is conducted in the presence of a catalyst system comprising chromium and ruthenium.

Thus, in one of its aspects, the invention is concerned with the decomposition of hydroperoxides.

The decomposition of hydroperoxide catalysed by transition metal complexes has been investigated for a number of years. It is generally assumed that in the first stage of decomposition, the hydroperoxide molecule forms an active complex with the metal catalyst. This complex decomposes subsequently into free radicals or molecular species which yield final products in further reactions.

As is well known in the art, the products of such decomposition reactions are employable as obtained as commercial products, for example, tertiary buty alcohol in the case of the decomposition of tertiary butyl hydroperoxide, or alternatively, may readily be converted by further reaction, as by oxidation, to derivatives thereof, for example, adipic acid, in the case of decomposition of cyclohexyl hydroperoxide producing a mixture of cyclohexanone and cyclohexanol.

In US Patent 3 879 467 there is disclosed a process for the catalytic oxidation of certain hydrocarbons utilising an organic hydroperoxide in the presence of a chromium catalyst to produce alcohols and ketones as the primary products. Also disclosed in this patent for this purpose are a number of other specific metal catalysts which resulted in low hydroperoxide conversions or low product yields, or both, or in instances where high conversions were noted, almost no product yields were obtained.

In US Patent 3 925 316, a process is disclosed for the preparation of mixture of cycloalkanols and cycloalkyl ketones by heating cycloalkyl hydroperoxides in the presence of, as catalyst, a soluble derivative of vanadium, molybdenum, or ruthenium.

Certain forms of ruthenium have also been reported in recent publications in connection with cumene hydroperoxide decomposition studies. In this regard, attention is directed to "Use of the Proton NMR Relaxation Method to Study the Coordination of Cumene Hydroperoxide With Cobalt and Ruthenium Carboxylates", V. M. Nekipelov, Dolk. Akad. Nauk SSSR, V 261 (6), 1377—81 (1981); "NMR Studies of .Mu3-Oxotriruthenium Hexacarboxylate Cumene Hydroperoxide Interaction", A. M. Trzeciak, Oxid. Commun., V. 1 (4), p. 295—303 (1980); "Cumene Hydroperoxide Decomposition Reaction Catalyzed by Ruthenium (III) beta.-diketonates", A. M. Trzeciak, et al, React. Kinet. Catal. Lett., V. 12 (1—2), p. 121—5 (1981); and "Decomposition of Organic Hydroperoxides on Ruthenium .pi.-Complexes", Yu A. Aleksandrov, Ah. Obshch. Khim., V. 48 (9), p. 2142 (1978).

Binary homogeneous metal catalyst combinations previously disclosed for decomposition of specific hydroxides include the combination of a particular salt of iron and copper in U.S. Patent 3,401,193, and an admixture comprised of cobalt and chromium compounds for effecting cyclohexane oxidation and decomposition of resultant hydroperoxide with the recovery of cyclohexanone and cyclohexanol in U.S. Patent 3,987,100.

The use of heterogeneous metal catalysts has also been disclosed in recently issued publications, for example, in U.S. Patent, 4,173,587, there is disclosed the use of a non-soluble rhenium compound for the decomposition of cumene hydroperoxide; in U.S. Patent 4,209,465, there is disclosed the decomposition of cumene hydroperoxide by use of specified carbonium, tropylium or oxonium salts as catalysts; and in U.S. Patent 4,059,598, there is disclosed from the decomposition of residual hydroperoxides resulting from propylene epoxidation in the presence of a homogeneous cobalt oxide catalyst which may also contain copper oxide as a promoter.

However, prior art catalyst systems were deemed unsatisfactory in their ability to provide a stable catalyst system or lacked desired activity in the decomposition of hydroperoxide, or failed to provide the selectivity to the desired product(s). In an effort to correct one or more of these apparent deficiencies, the trend has been to employ stabilizing ligands in combination with certain metallic catalysts; however, many of these ligands are not readily available and are expensive to employ in any large quantity, especially on a commercial scale.

It has now been discovered that hydroperoxides may be decomposed by use of a catalytically significant quantity of a binary catalyst system comprising an admixture of ruthenium and chromium, thereby eliminating the need for expensive and difficult to obtain stabilizing ligands.

Thus, according to the present invention, there is provided a process for the decomposition of a hydroperoxide, said process comprising contacting the hydroperoxide with a catalytic quantity of a catalyst system comprising chromium and ruthenium.

The catalyst system employed in the process of the present invention is characterized by its stability over extended periods of time in use while exhibiting improved activity for organic hydroperoxide decomposition and selectivity to the desired alcohol and ketone products in the case of organic hydroperoxide decomposition. Thus, the present invention comprises a process for the catalytic decomposition of a wide variety of hydroperoxides in high yield and selectivity to the desired products by employing a stable binary homogeneous catalyst system comprising ruthenium and chromium.

Although any hydroperoxide is deemed capable of decomposition in accordance with the process of the present invention, in general, typical hydroperoxides employable conform to the general formula:

R—OOH

wherein R is a member selected from the group consisting of hydrogen, a straight or branched chain alkyl or cycloalkyl group containing of from 2 to 15 carbon atoms, an aryl group such as a monocyclic or polycyclic group in which the cyclic groups may optionally be substituted with one or more substituents inert to the decompositon reaction, such as alkyl or alkoxy containing of from 1 to 7 carbon atoms, nitro, carboxyl or carboxyl ester containing up to 15 carbon atoms and a halogen atom such as chloride, bromide, or an alkaryl group in which the alkyl chain contains from 1 to 15 carbon atoms and the aryl group is as above described. Preferably, R is hydrogen, an alkyl or cycloalkyl group containing of from 4 to 12 carbon atoms or an alkaryl group in which the aromatic moiety is phenyl and the alkyl substituent is straight or branched chain alkyl or cycloalkyl containing up to about 6 carbon atoms. Illustrative preferred organic hydroperoxides employed as starting materials in the process of the present invention include tertiary butyl and isobutyl hydroperoxide, 2-methylbutyl-2 hydroperoxide, cyclohexyl hydroperoxide, α- and β-ethylbenzene hydroperoxide, cumene hydroperoxide, cyclohexylphenyl hydroperoxide and hydrogen peroxide.

The decomposition of the hydroperoxide in the presence of the binary homogeneous catalyst system of the present invention is carried out in known manner. In general, the reaction proceeds very rapidly over a wide variety of reaction conditions. Reaction temperatures employed may range from 25°C to 250°C, and preferably between 50°C and 150°C. In order to avoid the production of undesired by-products, the reaction temperature is not permitted to attain too high a level, thereby precluding thermal decomposition of the hydroperoxide. In addition, the products obtained by process of the present invention, other than the desired primary alcohol and ketone products, in the case of decomposition of an organic hydroperoxide, illustratively aldehydes, acids and peroxides, are removed during the decomposition reaction in order to reduce the possibility of undesired side reactions. Pressure at which decomposition is carried out is not critical and removal of such by-products may be effected by conducting the decomposition under reduced pressure. In general, atmospheric and superatmospheric pressure up to 150 bar (150 atmospheres) e.g. 0.1 to 150 bar (0.1 to 150 atmospheres), i.e. pressures sufficient to maintain the reactants in the liquid phase, may be conventionally employed.

The reaction time required for effecting completion of the decomposition of the hydroperoxide will depend on catalyst and hydroperoxide concentration and temperature. In general, decomposition of the organic hydroperoxide will be substantially completely effected in batch operation within 0.1 to 1000 minutes, preferably 1 to 60 minutes. In continuous operation, any convenient conversion rate may be adapted depending upon catalyst concentration and proportions, nature of of diluent, if employed, and consumption of liberated oxygen from hydroperoxide decomposition.

If desired, in order to more readily facilitate carrying out of the reaction, e.g. for recycle of the catalyst contained therein, the process of the present inention may be carried out in the presence of a diluent or solvent. Typical diluents employable in the process of the present invention are hydrocarbons from which the organic hydroperoxides may have been derived by oxidation, i.e. precursor starting materials, such as cyclohexane in the case where the desired hydroperoxide is cyclohexyl hydroperoxide, reaction by-products, such as acids and esters obtained in the reaction, or the alcohol which is the product of the reaction of the decomposition, for example, tertiary butyl alcohol in the case of the decomposition of tertiary butyl hydroperoxide. In the event the diluent employed is reactive under the reaction conditions of the process, either with the hydroperoxide or with liberated molecular oxygen, increased yields of the desired alcohol or ketone products are obtainable. If employed, the diluent is generally present in an amount between 10 and 98 percent, by weight, and preferably between 50 and 97 percent, by weight, based on the total weight of the reaction mixture. When such a diluent is employed, as will be appreciated by those skilled in the art, the reaction is effected at a rate and under reaction conditions of temperature and pressure sufficient to avoid excessive build-up of liberated oxygen, thereby precluding formation of a flammable composition in the vapor phase. Alternatively, if desired, the decomposition reaction may be carried out in the presence of an inert gas such as nitrogen or argon to dilute non-condensible gaseous products, primarily liberated oxygen, to avoid formation of a flammable gaseous mixture.

The binary catalyst system of the present invention may be homogeneous and comprise an admixture of ruthenium and chromium compounds which are soluble in the liquid hydroperpoxide to be decomposed or in the aforementioned diluent. Representative examples of ruthenium and chromium compounds employable in the process of the invention include ruthenium and chromium salts or carboxylic acids, salts or organic acids produced in the course of oxidation of the precursor hydrocarbon from which the organic hydroperoxide may have been obtained, carbonyls, sulfates, nitrates, halides, and organometallic compounds of those metals. Representative examples of ruthenium compounds include ruthenium naphthenate, ruthenium octoate, ruthenium laurate, ruthenium stearate, ruthenium linoleate, ruthenium acetylacetonate ruthenium nitrate, ruthenium chloride, ruthenium sulfate and ruthenium carbonyl. Representative examples of chromium compounds include chromium naphthenate, chromium octoate, chromium laurate, chromium palmitate, chromium stearate, chromium linoleate, chromium

acteylacetonate, chromium nitrate, chromium chloride, chromium sulfate and chromium carbonyl. Representative examples of organic acids which may be produced in the course of oxidation of the hydrocarbon starting material precursor to the organic hydroperoxide include: acetic, formic, propionic, isobutyric, caproic, valeric, adipic, glutaric, hydroxycaproic and benzoic acids.

As indicated, it was unexpectedly found in accordance with the process of the present invention, that a stable catalyst suitable for effecting decomposition of the hydroperoxide specified at high activity and selectivity to desired alcohols and ketones may be obtained by employing a relatively inexpensive promoting chromium-containing compound, together with an active ruthenium compound, without necessary of employment of expensive and difficult to obtain stabilizing ligands. The concentration of the catalyst in the liquid phase may vary widely. In general, the binary catalyst system of the present invention may advantageously be employed in amounts ranging from 0.01 ppm to 5,000 ppm of ruthenium and from 0.01 to 5,000 ppm of chromium, preferably 0.1 to 1,000 ppm of ruthenium and 0.1 to 1,000 ppm of chromium, each as metals. The upper limit of catalyst concentration appears to be dictated and controlled, however, more by economics in view of the relatively higher cost of ruthenium, and no particular advantages at the higher concentration stated are manifest. Since it is preferred to use lower quantities of catalyst in view of the cost of ruthenium metal, the stability of the catalyst system enhances the operating economics. Although these metals may be employed on an equal weight basis, in general, the proportion of chromium to ruthenium will range from about 0.1:1 to 10:1, and higher, in achieving the objectives of the process. In addition, it has been found, in accordance with the present invention, that a hydroperoxide concentration of at least 1%, and preferably of at least 3%, should be maintained during the decomposition reaction to maintain catalyst stability, i.e., preclude precipitation of at least a portion of the metal catalyst. If desired, the catalyst may also be employed in fixed-bed operation in known manner on a conventional carrier or in conjunction with a polymeric support such as a polyvinylpryidine, polypyrrolidone or polyphthalocyanine. Further, if desired, the catalyst system may optionally be employed with a ligand, although such operation is not required as above indicated.

As is apparent to those skilled in the art, the hydroperoxide decomposition may be effected in various manners. Hence, the hydroperoxide may "self decompose", in which case all of the alcohol and ketone moieties produced are derived directly from the hydroperoxides. Alternatively, in the event a diluent and/or solvent is employed in the decomposition reaction, the hydroperoxide may also decompose by a reaction involving the hydrocarbon diluent/solvent which is converted to alcohol and ketone products. Under these circumstances the process is represented as being the reaction of one mole of the hydrocarbon starting material with one mole of the corresponding hydroperoxide to yield two moles of alcohol and/or ketone. In addition, since there is a build-up of liberated molecular oxygen as a result of decomposition of hydroperoxide, additional oxidation of the hydrocarbon diluent/solvent may be anticipated as a result of reaction of such liberated oxygen therewith.

The reaction of the diluent/solvent hydrocarbon results in higher conversions, in general, of the hydrocarbon starting material to the useful oxidation products than will be realized if the hydroperoxide decomposed by itself. Of particular advantage would be to increase the amount of alcohol and ketone product derived from the diluent/solvent by its oxidation with the hydroperoxide or liberated oxygen, without adversely affecting the yield of alcohol and ketone derived directly from the intermediate hydroperoxide.

Accordingly, improvements in the oxidation step as well as in the decomposition step of the process for producing alcohols and ketones from hydrocarbon feed materials are highly desirable objectives.

In another of its aspects, therefore, the invention relates to an improved catalytic process for the production of alcohols and ketones wherein a hydrocarbon is oxidized in the presence of a molecular oxygen containing gas to produce a reaction mixture containing the corresponding hydroperoxide and the hydroperoxide is decomposed and either the oxidation or both the oxidation and the decomposition take place in the presence of the catalyst composition comprising chromium and ruthenium and the products of the decomposition generally comprise alcohol and ketone.

The oxidation of aliphatic and alicyclic hydrocarbons to end product alcohols and ketones, optionally via hydroperoxide decomposition, is a well-known competitive, large-volume industrial practice. Experience in the operation of such processes, which are reflected in the disclosures of numerous patents and literature references, has indicated that the oxidation must be carried out at controlled conversion levels, for example, to minimize the formation of other undesirable oxidation products, some of which may have an adverse effect on acceptability in industrial usage directly or may have deleterious effects in the production of derivative products and/or of purity of the derivative products produced. Relatively minor process improvements, such as in the yield of the intermediate hydroperoxide, or in the conversion of the hydroperoxide to desired alcohol and ketone product, may result in highly beneficial cost advantages. Accordingly, there is a strong economic incentive to increase the efficiency of the oxidation process from which such products are obtained.

US Patent 3 530 185 discloses a process for the partial oxidation of cyclohexane employing a mixture of gases including molecular oxygen at controlled partial pressure and inert gas in the presence of a catalyst, such as a cobalt compound, which will cause decomposition of the intermediate cyclohexyl hydroperoxide to cyclohexanol and cylochexanone.

Other publications of relevance are those referred to above in connection with the prior art relating to

the decomposition of hydroperoxides.

Thus, further in accordance with the present invention there is provided an improvement in the process for producing alcohols and ketones wherein hydrocarbon feed material, such as aliphatic and alicyclic hydrocarbons, is oxidized in the presence of a molecular oxygen-containing gas to provide a reaction mixture containing the corresponding hydroperoxide and the hydroperoxide is decomposed in the presence of starting hydrocarbon feed to provide a mixture containing the desired alcohol and ketone products, the improvement comprising effecting the oxidation reaction by contacting the hydrocarbon feed material, illustratively an aliphatic or an alicyclic hydrocarbon, with a molecular oxygen containing gas, such as air, preferably at a temperature of from 50°C to 250°C, more preferably between 75°C and 225°C, in the presence of a catalytic quantity of a catalyst system comprising an admixture of ruthenium and chromium, thereby forming various oxidation reaction products, including the corresponding hydroperoxide of said hydrocarbon feed, alcohols, ketones, etc., and, optionally, conducting decomposition of the hydroperoxide by contacting a reaction mixture containing such hydroperoxide in the presence of a diluent, preferably unreacted hydrocarbon, with a catalytic amount of said catalyst system preferably at a temperature of from 25°C to 250°C, and more preferably between 50°C and 150°C, and optionally in the presence of a molecular oxygen.

The advantages realized by use of the catalyst described, as compared with the use of chromium or ruthenium alone, include improved conversions of the hydrocarbon, starting material to oxidation products, and of hydroperoxide to alcohol and ketone products; improved activity for organic hydroperoxide decomposition and selectivity to the desired alcohol and ketone products; and maintenance of stability of the catalyst systems, as evidenced by substantial elimination of the formation of insoluble metal-containing compositions.

The catalyst system employed in this process comprises an admixture of ruthenium and chromium compounds which are preferably soluble or capable of being solubilized in the hydrocarbon feed to be oxidized, as well as in the hydroperoxide intermediate to be decomposed, i.e. the reaction mixture. Representative examples of ruthenium and chromium compounds employable in the process are listed above.

The hydrocarbon feed materials employed in the process of the present invention comprise both aliphatic and alicyclic hydrocarbons. Suitable aliphatic compounds include both paraffinic and olefinic hydrocarbons, such as propylene, n-butane, isobutane, isobutylene, l-butene, and hexadecane, while the alicyclic compounds include both saturated polycylic ring compounds such as decalin and non-aromatic unsaturated and saturated ring compounds such as cyclohexene and cyclohexane. These compounds may be substituted by groups which would be inert to oxidation under conditions of the process, as for example, nitro, nitrile, phenyl, sulfone and carboxylic acid groups. In general, the aliphatic and alicyclic hydrocarbons suitable for feed reactant in the process of the present invention contain from 2 to 25 carbon atoms and preferably 3 to 10 carbon atoms. It is noted that in the saturated compounds, the secondary and/or tertiary carbon atoms are more readily oxidized in the process, while in unsaturated hydrocarbons, those carbon atoms which are adjacent to a double bond, whether they be primary, secondary or tertiary, will be readily oxidized. As previously indicated, processes for the oxidation of hydrocarbons contemplated herein are well described in the litarature. The process of the present invention may be employed in such a manner as to carry out the oxidation and/or decomposition reactions only, or in combination, sequentially and/or in series. Hence, for example, hydrocarbon oxidation and corresponding hydrocarbon hydroperoxide decomposition may be conducted in separate stages or in the same stage, regardless of whether or not the oxidation is conducted in a series of zones. It is to be understood that, expect for details sepcified, the oxidation and decomposition steps are effected in conventional manner.

The distribution of the products from the oxidation of the hydrocarbon feed depends upon both temperature and percent conversion of the hydrocarbon to products. Thus, within the given temperature range indicated, in general, the lower the temperature, the greater the amount of hydroperoxide which will be formed as the principle product. As the temperature is increased, it is found that the oxidation of primary carbon atoms leads principally to the production of by-product aldehydes and acids; the oxidation of secondary carbon atoms leads principally to the production of ketones and alcohols; while the oxidation of tertiary carbon atoms generally results either in decomposition of the hydroperoxide to form an alcohol, or in chain scission to yield a ketone and an alcohol as the principal products.

Oxidation reaction conditions for production of desired product depend on factors such as the particular catalyst, conversion desired, reaction temperature and time, as well as upon the starting material employed. Thus, for example, the oxidation of isobutane in accordance with the present invention at temperatures between 100°C and 160°C will provide the corresponding hydroperoxide, while the same oxidation carried out at more elevated tempratures of between 190°C and 250°C will result in acetone and methyl alcohol as the principal products.

The oxidation reaction of the process of the present invention, utilizing the aforedescribed catalyst system, is conveniently carried out by the rapid passage of a molecular oxygen containing gas, such as air, through a suitable reactor, to which there has been charged a mixture of the hydrocarbon substrate and catalyst system. The molecular oxygen containing gas is brought into intimate contact with the liquid phase, for example, by the use of high-speed stirrers, nozzles, or the like in conventional manner. The concentration of the catalyst system in the liquid phase, in general, may vary widely, depending upon the

nature and amount of material to be oxidized. In general, however, the quantity of catalyst employed in the oxidation step will vary from 0.01 to 1000 ppm of metal (or mixed metal), and greater, preferably from 1 to 100 ppm, by weight, in the total mixture; the catalyst concentration is also dependent upon temperature and conversion desired. Moreover, with respect to the nature of catalyst employed, the proportion of chromium to ruthenium may range from 0.5:1 to 10:1 and higher, in achieving the objectives of the process. If desired, pressures from 1 to 100 bar (1 to 100 atmospheres), preferably between 2 and 50 bar (2 and 50 atmospheres), may be employed to maintain the liquid phase.

To moderate the decomposition reaction, the process is usually carried out in a suitable diluent/solvent. Suitable diluents include alkanes, such as hexane, heptane and hexane; cycloalkanes, such as cyclopentane, methyl cyclopentane, and cyclohexane; aromatic hydrocarbons, such as benzene, toluene, and xylene; and mixtures of such hydrocarbons with aromatic halides or ethers such as chlorobenzene or diphenyl ether. To facilitate effecting the reaction, the solvent employed is the hydrocarbon from which the hydroperoxide may have been derived by oxidation, i.e. precursor starting materials, for example, such as cyclohexane in the case where the desired hydroperoxide is cyclohexyl hydroperoxide.

The rate of input of molecular oxygen containing gas will depend upon the temperature and pressure utilized during the oxidation period, and heat removal limitations since these oxidations are highly exothermic. Normally there is provided at least an amount theoretically sufficient substantially to convert the starting material to the corresponding oxidation product, and preferably, in slight excess of this amount. As previously indicated molecular oxygen is also obtained as liberated oxygen resulting from the decomposition reaction to be conducted. It has been found that a flow rate ranging from 10 to 1,000 liters per liter of solution per hour is generally sufficient for most conversions. Any unreacted oxygen may be recycled to the reactor.

The reaction is generally complete in from 1 to 10 hours, depending upon the amount of substrate employed, particularly catalyst, conversion desired and reaction temperature employed. Preferably, the reaction is terminated after a period of 1 to 4 hours and the products are separated from the reactants by conventional means such as by distillation. The catalyst retained in the distill and also may be recovered in conventional manner or recycled to the oxidation reaction.

If desired, small amounts of hydroperoxide may be introduced into the oxidation reaction medium to act as reaction initiator. Thus, for example, when isobutane is being oxidized, minor amounts, up to 3%, by weight of the reaction mixture of tertiary butyl hydroperoxide may be added in order to initiate the reaction.

The concentration of hydroperoxide in the reaction mixture obtained from the oxidation reaction, i.e. hydroperoxide feed of the decomposition reaction, may vary widely and, in general, ranges from 1% to 80%, by weight and preferably, from 2% to 60% by weight, based on the total reaction mixture. The catalyst concentration in the total mixture will normally range from 0.01 up to 5,000 ppm of metal and greater, and preferably from 1 to 100 ppm. Lower catalyst concentrations are employed normally at higher temperatures. The reaction time will depend on the temperature and the catalyst concentration and will usually range from 0.1 to 5 hours, preferably from 0.1 to 2 hours. Although longer reaction times may be employed, in general no particular advantage is achieved thereby. Generally, the temperature in the decomposition reaction of the process of the invention will range from 20° to 200°C, preferably from 50° to 150°C. Pressures of from 1 to 100 bar (1 to 100 atmospheres) preferably of from 2 to 50 bar (2 to 50 atmospheres) may be employed in order to maintain the reaction in liquid phase.

Although the hydroperoxide and hydrocarbon starting material/solvent will be supplied to the hydroperoxide decomposition reactor as the liquid effluent obtained from the primary oxidation zone, it should be appreciated that this reaction mixture will also contain final desired alcohol and ketone products, along with minor amounts of other oxidation products and catalyst remaining from the oxidation reaction. However, if desired, a solution of pure hydroperoxide and solvent hydrocarbon may also be employed as the starting material for the decomposition step.

Decomposition of hydroperoxide may be effected in the presence or absence of added molecular oxygen. It is to be appreciated that oxygen may additionally be desired as liberated oxygen as a result of decomposition of previously formed hydroperoxide. It has also been found that when the hydroperoxide is decomposed in a stage separate from oxidation and at a lower temperature, the yield of alcohol and ketone from hydrocarbon participation in the decomposition of the hydroperoxide may be increased, without significantly reducing the yield of alcohol and ketone obtained directly from hydroperoxide decomposition, simply by adding molecular oxygen to the decomposition reaction mixture. Alternatively, the hydroperoxide decomposition may be effected at an increased rate to yield excessive quantities of liberated molecular oxygen which will provide the same result as the addition of molecular oxygen, as previously explained. The introduction of oxygen may be generally carried out in any convenient manner to permit control of the concentration of oxygen desired. Hence, oxygen may be supplied as air, as air enriched with added oxygen, or as air or molecular oxygen diluted with an inert gas such as nitrogen or argon.

Any amount of added oxygen will bring about increased production of alcohol and ketone products via hydrocarbon oxidation. The mole ratio of oxygen to hydroperoxide may also vary widely and generally will range from 0.1 to 20. The preferred range of oxygen/hydroperoxide mole ratio will range from 0.5 to 5 at which significant increases may be noted.

The time required for the decomposition reaction in the presence of molecular oxygen will depend on

the hydroperoxide concentration, the nature of catalyst, and catalyst concentration, the oxygen/hydroperoxide mole ratio and the reaction temperature. Normally the reaction time will vary between 0.1 hours and 5 hours and preferably between 0.1 hours and 2 hours. When oxygen is used during the decomposition step, the temperature will vary from 50° to 250°C and will preferably range from 85° to 150°C.

The process of the present invention may be carried out by effecting the oxidation and decomposition steps concurrently in the same stage or the oxidation and decomposition reactions may be carried out in separate stages. The process of the invention may be effected by a batch method or continuous method, although it is preferred to operate the process in a continuous manner in the presence of added molecular oxygen, as described above.

The invention is now illustrated by the following examples in which all temperatures are in degrees Centigrade and all percentages are by weight unless othewise specified. Examples I to XVI and XIX to XXIV illustrate the decomposition of hydroperoxide in accordance with the invention and Examples XVII and XVIII illustrate the production of oxidation products by oxidation of hydrocarbon to hydroperoxide and decomposition of the hydroperoxide.

Examples I—VI

To a 100 ml flask equipped with a condenser and a side arm capped with a rubber septum for liquid sampling, there is charged 100 ppm of the metal or mixture of metals as their salts identified in Table I, below, solubilised in 40 cc of an oxidation reaction product (Reactant Feed) obtained from the non-catalysed molecular oxygen oxidation of isobutane containing 42 parts of tertiary butyl hydroperoxide (TBHP), 56 parts of tertiary butyl alcohol (TBA) and 2 parts of a crude mixture comprised of low molecular weight oxygen-containing liquids. The flask and contents are rapidly heated to reflux temperature, about 78°C, by immersing in an oil bath, and held at such temperature for a period of 2 hours. Liquid samples are withdrawn after the indicated period and analyzed by gas-liquid chromatography. Table I sets forth the results of the decomposition reaction.

TABLE I

| Ex. No. | Metal Salt Catalyst | TBHP Decomposition wt(%) | Physical Characteristics of Reaction Product |
|---|---|---|---|
| I | Chromium (III) Acetyl-acetonate | 4.3 | clear, deep yellow solution |
| II | Cobalt (II) Acetyl-acetonate | 37.4 | turbid, pink solution containing precipitated metal |
| III | Manganese (II) Acetylacetonate | 16.9 | turbid, pinkish solution containing white precipitate |
| IV | Ruthenium (III) Acetylacetonate | 99.0 | turbid, green solution containing grey precipitate |
| V | Equimolar mixture of: Ruthenium (III) and Chromium (III) Acetylacetonàtes | 96.6 | clear yellow solution with no precipitate |
| VI | Equimolar mixture of: Cobalt (II) and Chromium (III) Acetylacetonates | 48.1 | clear yellow solution |

The results of the experiments set forth in Table I, above, demonstrate the superior activity of ruthenium for decomposition of tertiary butyl hydroperoxide and that equimolar admixtures of ruthenium and chromium provide a stable catalyst system for the decomposition of hydroperoxides while maintaining high activity for the decomposition reaction.

Examples VII—XI

The examples set forth below demonstrate continuous operation of the process of the invention.

The apparatus employed is a glass reactor having a volume of approximately 50 cc equipped with a stirrer, a side-arm used to feed the hydroperoxide containing solution, a glass thermocouple well and an additional side arm for removal of the alcohol product as a vapor. Nitrogen is fed at a rate of 85 cc per minute to the reactor through the inlet side arm to dilute the non-condensable product, primarily oxygen. The reactor is glass-jacketed and equipped with inlet and outlet arms to permit a continuous circulation of oil from a heating bath to maintain the reaction temperature at reflux temperature.

The reactor is charged with 10 grams of the Reactant Feed described in Example I above, having the catalyst or catalyst mixture indicated in Table II below, solubilized therein. Reactant Feed is metered continuously from a graduated reservoir over the period indicated. The vapor product from the reactor is cooled by passing over a condenser maintained at 25°C. The liquid condensate is analyzed by gas-liquid chromatography for tertiary butyl alcohol (TBA), acetone, methanol, ditertiary butyl peroxide (DTBP) and tertiary butyl hydroperoxide (TBHP). The non-condensible gases are monitored by gas chromatography for carbon dioxide, carbon monoxide, methane, oxygen and nitrogen. The results obtained are set forth in Table II, below:

TABLE II

| Example No. | Catalyst[1] (ppm) | Average Rate of TBHP Decomposition[2] | Time[3] | Product Selectivity (%) TBA | Methanol | DTBP | Acetone | Physical Characteristics of Reaction Products |
|---|---|---|---|---|---|---|---|---|
| VII | Ruthenium III (100) | 31,700 | 6 | 83.6 | 2.7 | 3.8 | 9.9 | turbid solution containing grey precipitate |
| VIII | Ruthenium III (10) and Chromium III (10) | 89,900 | 6 | 90.4 | 1.1 | 4.7 | 3.8 | clear solution |
| IX | Ruthenium III (100) and Chromium III (50) | 7,900 | 16 | 91.6 | 0.8 | 4.4 | 3.2 | clear solution |
| X | Cobalt II (100) and Chromium III (100) | 5,300 | 5 | 92.2 | 0.5 | 4.6 | 2.7 | clear solution |
| XI | Ruthenium III (25) and Chromium III (25) | 43,875 | 116 | 86.1 | 2.9 | 4.2 | 6.8 | clear solution |

[1]present as the acetylacetonate salt in 10 grams of Reactant Feed

[2]grams of TBHP decomposed/gram of metal catalyst/hour

[3]hours of continuous operation

The results of the above experiments set forth in Table II demonstrate that ruthenium exhibits high activity for continuous hydroperoxide decomposition and that chromium exhibits a stabilizing effect over extended periods of time on ruthenium during the decomposition reaction while maintaining high activity and selectivity to desired tertiary butyl alcohol product.

Example XII

Into a 300 cc stainless steel stirred reactor, there is charged 58 parts of isobutane and the reactor is heated to a temperature of 111°C. Thereafter, 100 parts of the Reactant Feed described in Example I above, is premixed with a binary catalyst comprised of 3 ppm of ruthenium III acetylacetonate and 3 ppm of chromium III acetylacetonate and the resultant admixture is added to the reactor under 81.6 to 88.4 bar g (1200 to 1300 psig) nitrogen pressure. The reaction is conducted isothermically over a period of 2.5 hours at which point analysis of the reaction products is made by gas-liquid chromatography. The results indicate a tertiary butyl hydroperoxide conversion of 98%, with molar selectivities to: tertiary butyl alcohol of 85.6; acetone 7.5%; ditertiarybutyl peroxide of 5.5%; methyl ethyl ketone of 0.1%; and unidentified by-products of 1.3%. Incorporation of liberated molecular oxygen into the products of the reaction of 72.4% is obtained.

This Example demonstrates that a mixture of soluble ruthenium and chromium constitutes a very active and stable catalyst for decomposition of TBHP in the presence of isobutane. Under the operating conditions employed, the active oxygen of decomposed TBHP can be substantially consumed to produce products other than molecular oxygen, thereby significantly reducing costs associated with dilution and recycle or disposal of large quantities of liberated oxygen.

Example XIII

The same procedure is followed as in Example I, except that ethyl benzene hydroperoxide and ethyl benzene are employed as the hydroperoxide and diluent, respectively, and a catalyst system comprised of 50 ppm of ruthenium and 50 ppm of chromium, each in the trivalent state and present as the acetylacetonate salt, is employed.

The results indicate that 97% of the hydroperoxide decomposes and quantitative analysis, by gas-liquid chromatography, of the resulting crude products shows a yield of α methylbenzyl alcohol and acetophenone of 92%, based on the hydroperoxide feed. The reaction product is a clear solution with no detectable quantities of any precipitate, indicating stability of the catalyst.

Example XIV

The same procedure is followed as in Example I, except that cumene hydroperoxide and cumene are employed as the hydroperoxide and diluent, respectively, and a catalyst system comprised of 50 ppm of ruthenium and 50 ppm of chromium each in the trivalent state and present as the acetylacetonate salt, is employed.

The results indicate that 98% of the hydroperoxide decomposes and quantitative analysis, by gas-liquid chromatography, of the resulting crude products shows a yield of phenol of 95% based on the hydroperoxide feed. The reaction product is a clear solution with no detectable quantities of any precipitate, indicating stability of the catalyst.

Example XV

The same procedure is followed in Example I, except that cyclohexylbenzene hydroperoxide and cyclohexane are employed as the hydroperoxide and diluent, respectively and a catalyst system comprised of 10 ppm of ruthenium and 50 ppm of chromium, each in the trivalent state and present as the acetylacetonate salt, is employed.

The results indicate that 99% of the hydroperoxide decomposes and quantitative analysis by gas-liquid chromatography, of the resulting crude products shows a yield of phenol and cyclohexanone of 89%, based on the hydroperoxide feed. The reaction product is a clear solution with no detectable quantities of any precipitate, indicating stability of the catalyst.

Example XVI

The same procedure is followed as in Example I, except that cyclohexyl hydroperoxide and cyclohexane are employed as the hydroperoxide and diluent, respectively, and a catalyst system comprised of 20 ppm of ruthenium and 50 ppm of chromium, each in the trivalent state and present as the acetylacetonate salt, is employed.

The results indicate that 97% of the hydroperoxide decomposes and quantitative analysis, by gas-liquid chromatography, of the resulting crude products shows a yield of cyclohexanone and cyclohexanol of 84%, based on the hydroperoxide feed. The reaction product is a clear solution with no detectable quantities of any precipitate, indicating stability of the catalyst.

### Example XVII

The apparatus to be used for effecting oxidation consists of a stainless steel autoclave having a volume of approximately 250 cc and usable at internal pressures up to about 340 bar g (5000 psig) equipped with a magnetic stirrer, an inlet tube attached to a pressure gauge, an outlet tube for liquid samples and an outlet tube for gas samples. The autoclave is equipped with a pressure-relief valve and heating is provided externally by means of a reactor jacket connected to an oil pump/bath regulated by a heater control. Internal cooling coils are provided to quench the reaction contents at the end of the run or in the event the reaction exothermed. Temperatures are measured with a resistance thermometer.

The reactor is charged with 10 ppm of an equimolar mixture of ruthenium acetylacetonate and chromium acetylacetonate in 150 cc of isobutane at about 50 bar (50 atmospheres) of air flowing continuously at about 10 litres/hour (stp). With continuous stirring throughout the run, the contents of the reactor are brought to a temperature of about 150°, at which point evidence of an exothermic reaction is noted. After about 300 mins. when the molecular oxygen consumption indicates that about 20% of the isobutene is converted to products, the reactor is cooled and opened for recovery of the products thereof. Gas liquid analysis of the liquid reaction product shows that the major product is tertiary butyl alcohol with minor amounts of tertiary butyl hydroperoxide, methyl alcohol, ditertiary butyl peroxide and acetone.

### Example XVIIA (Comparative)

Control Experiment with Ruthenium Acetylacetonate

The procedure of Example XVIIA is repeated except that 10 ppm of ruthenium acetylacetonate is used. A precipitate is found in the reactor after completion of the run indicating that a good portion of the ruthenium precipitates. Gas liquid chromatography analysis of the reaction product shows that only about 60% of the tertiary butyl hydroperoxide produced is decomposed in the control run with ruthenium acetylacetonate, compared with essentially complete decomposition when the mixture of ruthenium and chromium acetylacetonates is used.

The results of this experiment show that the mixture of ruthenium and chromium acetylacetonates is a better catalyst then ruthenium acetylacetonate alone for the decomposition of tertiary butyl hydroperoxide and that the mixture of ruthenium and chromium acetylacetonates is a stable catalyst system for effecting the decomposition reaction.

### Example XVIII

The reactor employed in Example XVII is charged with 100 cc of cyclohexane, 10 ppm of a equimolar mixture ruthenium acetylacetonate and chromium acetylacetonate. The reactor is sealed and the reaction mixture is heated at 140° for 300 minutes with stirring to obtain a product mixture which, when analyzed by gas liquid chromatograph, shows the presence of 65% of cyclohexanone and 25% of cyclohexanol. The concentration of cyclohexyl hydroperoxide is 4%, corresponding to a practically complete conversion of the cyclic $C_6$ moiety of cyclohexyl hydroperoxide to cyclohexanone and cyclohexanol.

### Examples XIX—XXIV

Employing the reactor described in Example XVII various catalysts of the invention are used to decompose cyclohexyl hydroperoxide prepared by oxidizing cyclohexane as in Example XVIII without the decomposition catalyst being present during the oxidation. The reactor is pressurized with nitrogen to 34 bar g (500 psig) to minimize the vaporization of cyclohexane and dilute the liberated oxygen. The results and details of these examples are set forth in Table III below.

TABLE III

| Example | Catalyst | ppm | Temperature | Hydroperoxide Decomposed After 120 minutes |
|---|---|---|---|---|
| XIX | chromium octoate and ruthenium octoate | 10<br>10 | 80 | 97 |
| XX | chromium octoate and ruthenium octoate | 10<br>3 | 120 | 99 |
| XXI | chromium laurate and ruthenium laurate | 20<br>20 | 70 | 95 |
| XXII | chromium laurate and ruthenium laurate | 20<br>5 | 100 | 98 |
| XXIII | chromium stearate and ruthenium stearate | 10<br>10 | 80 | 96 |
| XXIV | chromium stearate and ruthenium stearate | 50<br>50 | 60 | 94 |

## Claims

1. A process for the decomposition of a hydroperoxide said process comprising contacting the hydroperoxide with a catalytic quantity of a catalyst system comprising chromium and ruthenium.

2. A process as claimed in claim 1 wherein the catalyst system is present in an amount of from 0.01 ppm to 5000 ppm of ruthenium and of from 0.01 ppm to 5000 ppm of chromium.

3. A process as claimed in claim 1 or claim 2 wherein the decomposition reaction is effected in the presence of a diluent.

4. A process as claimed in claim 3 wherein the diluent is a member selected from the group consisting of isobutane, tertiary butyl alcohol, ethyl benzene, cyclohexane and acetic acid.

5. A process as claimed in claim 3 or claim 4 wherein a hydroperoxide concentration of at least about 1 percent is maintained during the composition reaction.

6. A process as claimed in any one of claims 1 to 5 wherein the hydroperoxide is of the formula:

ROOH

wherein R is selected from hydrogen, straight and branched chain alkyl groups containing from 2 to 15 carbon atoms, aryl groups optionally substituted with one or more substituents inert to the decomposition reaction and selected from alkyl and alkoxy groups of from 1 to 7 carbon atoms, nitro, carboxyl and carboxyl ester containing up to 15 carbon atoms, halo-, and alkaryl radicals in which the alkyl chain contains of from 1 to 15 carbon atoms.

7. A process as claimed in any one of claims 1 to 6 wherein the hydroperoxide is selected from tertiary butyl hydroperoxide, cyclohexyl hydroperoxide, cumene hydroperoxide, ethylbenzene hydroperoxide and cyclohexylphenyl hydroperoxide.

8. A process as claimed in any one of claims 1 to 7 wherein the ruthenium and chromium catalyst is present in the form of at least one salt of a carboxylic acid or the catalyst comprises an admixture of nitrates or sulfates of ruthenium and chromium.

9. A process as claimed in any one of claims 1 to 8 wherein the decomposition reaction is effected at temperatures between 25°C and 250°C and a pressure of between 0.1 and 150 bar (0.1 and 150 atmospheres).

10. A process for producing a mixture containing decomposition products of an organic hydroperoxide wherein a hydrocarbon is oxidised in the presence of a molecular oxygen containing gas to provide a reaction mixture containing the corresponding hydroperoxide of said hydrocarbon and said hydroperoxide is decomposed in the presence of starting hydrocarbon to provide a mixture containing reaction products of such decomposition reaction, including alcohol and/or ketone products, characterised in that the oxidation reaction is conducted by contacting the hydrocarbon with a molecular oxygen containing gas in the presence of a catalytic amount of a catalyst system comprising chromium and ruthenium and the decomposition reaction is conducted by contacting a reaction mixture containing the hydroperoxide of the hydrocarbon starting material in the presence of a diluent with a catalytic amount of said catalyst system, optionally in the presence of a molecular oxygen containing gas.

11. A process as claimed in claim 10 wherein the diluent is the hydrocarbon subjected to oxidation in the oxidation step of the reaction.

12. A process as claimed in claim 10 or claim 11 wherein the reaction is effected at a temperature of between 50°C and 250°C.

13. A process for the oxidation of a hydrocarbon to produce a hydroperoxide which comprises contacting said hydrocarbon with a molecular oxygen containing gas at a temperature of from 50°C to 250°C in the presence of a catalytic quantity of a catalyst system comprising chromium and ruthenium.

14. A process as claimed in any one of claims 10 to 13 wherein the catalyst system present in the oxidation step is in an amount of form 0.01 ppm to 1,000 ppm ruthenium and from 0.01 ppm to 1,000 ppm of chromium.

15. A process as claimed in any one of claims 1 to 14 wherein the ruthenium and chromium are present as compounds soluble in the reaction mixture.

16. A process as claimed in any one of claims 10 to 15 wherein the ruthenium and chromium catalyst is present in the form of at least one salt of a carboxylic acid or the ruthenium and chromium are present in the form of nitrate or acetate salts.

17. A process as claimed in any one of claims 10 to 16 wherein the hydrocarbon is selected from isobutane, cyclohexane, cumene and ethylbenzene.

**Patentansprüche**

1. Verfahren zum Abbau eines Hydroperoxids, bei dem man das Hydroperoxid mit einer katalytischen Menge eines Katalysatorsystems in Kontakt bringt, das Chrom und Ruthenium enthält.

2. Verfahren nach Anspruch 1, worin das Katalysatorsystem in einer Menge von 0,01 ppm bis 5000 ppm Ruthenium und 0,01 ppm bis 5000 ppm Chrom vorhanden ist.

3. Verfahren nach Anspruch 1 oder 2, worin die Abbaureaktion in Gegenwart eines Verdünnungsmittels durchgeführt wird.

4. Verfahren nach Anspruch 3, worin das Verdünnungsmittel aus der Gruppe bestehend aus Isobutan, tert. Butylalkohol, Ethylbenzol, Cyclohexan und Essigsäure ausgewählt wird.

5. Verfahren nach Anspruch 3 oder 4, worin eine Hydroperoxidkonzentration von mindestens etwa 1% während der Abbaureaktion aufrechterhalten wird.

6. Verfahren nach einem der Ansprüche 1 bis 5, worin das Hydroperoxid die Formel:

ROOH

aufweist, worin R ausgewählt ist aus Wasserstoff, geradkettigen und verzweigtkettigen Alkylgruppen mit 2 bis 15 Kohlenstoffatomen, Arylgruppen, die gegebenenfalls mit einem oder mehreren Substituenten, die inert für die Abbaureaktion sind, substituiert sind, und die aus Alkyl- und Alkoxygruppen mit 1 bis 7 Kohlenstoffatomen, Nitro-, Carboxyl- und Carboxylester mit bis zu 15 Kohlenstoffatomen, Halo- und Alkarylresten, in denen die Akylkette 1 bis 15 Kohlenstoffatome enathält, ausgewählt sind.

7. Verfahren nach einem der Ansprüche 1 bis 6, worin das Hydropeoxid aus tert. Butylhydroperoxid, Cyclohexylhydroperoxid, Cumenhydroperoxid, Ethylbenzolhydroperoxid und Cyclohexylphenylhydroperoxid ausgewählt wird.

8. Verfahren nach einem der Ansprüche 1 bis 7, worin der Rutheniunmm- und Chromkatalysator in Form mindestens eines Salzes einer Carbonsäure vorhanden ist, oder der Katalysator eine Mischung von Nitraten oder Sulfaten von Ruthenium und Chrom enthält.

9. Verfahren nach einem der Ansprüche 1 bis 8, worin die Abbaureaktion bei Temperaturen zwischen 25°C und 250°C und einem Druck zwischen 0,1 und 150 bar (0,1 und 150 Atmosphären) durchgeführt wird.

10. Verfahren zur Herstellung einer Mischung, die Abbauprodukte eines organischen Hydroperoxids enthält, worin ein Kohlenwasserstoff in Gegenwart eines molekularen Sauerstoff enthaltendem Gas oxidiert wird, um eine Reaktionsmischung zu ergeben, die das entsprechende Hydroperoxid des Kohlenwasserstoffs enthält, und worin das Hydroperoxid in Gegenwart des Ausgangskohlenwasserstoffs abgebaut wird, um eine Mischung zu ergeben, die Reaktionsprodukte einer solchen Abbaureaktion, einschließlich Alkohol und/oder Ketonprodukte enthält, dadurch gekennzeichnet, daß man die Oxidationsreaktion durch Inkontaktbringen des Kohlenwasserstoffs mit einem molekularen Sauerstoff enthaltendem Gas in Gegenwart einer katalytischen Menge eines Katalysatorsystems, das Chrom und Ruthenium enthält, durchführt und die Abbaureaktion durch Inkontaktbringen einer Reaktionsmischung, die das Hydroperoxid des Kohlenwasserstoff-Ausgangsmaterials enthält, in Gegenwart eines Verdünnungsmittels mit einer katalytischen Menge des Katalysatorsystems, gegebenenfalls in Gegenwart eines molekularen Sauerstoff enthaltendem Gas, durchführt.

11. Verfahren nach Anspruch 10, worin das Verdünnungsmittel der Kohlenwasserstoff ist, der der Oxidation im Oxidationsschritt der Reaktion unterworfen wird.

12. Verfahren nach Anspruch 10 oder 11, worin die Reaktion bei einer Temperatur zwischen 50°C und 250°C durgeführt wird.

13. Verfahren zur Oxidation eines Kohlenwasserstoffes zur Herstellung eines Hydroperoxids, bei dem man den Kohlenwasserstoff mit einem molekularen Sauerstoff enthaltendem Gas bei einer Temperatur von 50°C bis 250°C in Gegenwart einer katalytischen Menge eines Katalysatorsystems, das Chrom und Ruthenium enthält, in Kontakt bringt.

14. Verfahren nach einem der Ansprüche 10 bis 13, worin die Menge des im Oxidationsschritt vorhandenen Katalysatorsystems 0,01 ppm bis 1000 ppm Ruthenium und 0,01 ppm bis 1000 ppm Chrom beträgt.

15. Verfahren nach einem der Ansprüche 1 bis 14, worin das Ruthenium und Chrom als in der Reaktionsmischung lösliche Verbindungen vorliegen.

16. Verfahren nach einem der Ansprüche 10 bis 15, worin der Ruthenium und Chromkatalysator in Form mindestens eines Salzes einer Carbonsäure vorhanden ist, oder das Ruthenium und Chrom in Form von Nitrat- oder Acetatsalzen vorliegen.

17. Verfahren nach einem der Ansprüche 10 bis 16, worin der Kohlenwasserstoff aus Isobutan, Cyclohexan, Cumen und Ethylbenzol ausgewählt wird.

**Revendications**

1. Procédé pour la décomposition d'un hydroperoxyde, caractérisé en ce qu'il comprend la mise en contact de cet hydroperoxyde avec une quantité catalytique d'un système catalyseur comprenant du chrome et du ruthénium.

2. Procédé suivant la revendication 1, caractérisé en ce que le système catalyseur est présent en une quantité de 0,01 ppm à 5000 ppm de ruthénium et de 0,01 ppm à 5000 ppm de chrome.

3. Procédé suivant la revendication 1 ou la revendication 2, caractérisé en ce que la réaction de décomposition est effectuée en présence d'un diluant.

4. Procédé suivant la revendication 3, caractérisé en ce que le diluant est choisi dans le groupe comprenant l'isobutane, l'alcool t-butylique, l'éthylbenzène, le cyclohexane et l'acide acétique.

5. Procédé suivant la revendication 3 ou la revendication 4, caractérisé en ce qu'une concentration d'hydroperoxyde d'au moins environ 1% est maintenue pendant la réaction de décomposition.

6. Procédé suivant l'une quelconque des revendications 1 à 5, caractérisé en ce que l'hydroperoxyde est représenté par la formule:

$$ROOH$$

dans laquelle R est choisi parmi un atome d'hydrogène, des groupes alcoyles à chaîne droite ou ramifiée contenant 2 à 15 atomes de carbone, des groupes aryles éventuellement substitués avec un ou plusieurs substituants inertes pour la réaction de décomposition et choisis parmi des groupes alcoyles et alcoxy de 1 à 7 atomes de carbone, nitro, carboxy et ester carboxylique contenant jusqu'à 15 atomes de carbone, halo-, et des radicaux alcaryles dans lesquels la chaîne alcoyle contient de 1 à 15 atomes de carbone.

7. Procédé suivant l'une quelconque des revendications 1 à 6, caractérisé en ce que l'hydroperoxyde est choisi parmi l'hydroperoxyde de t-butgyle, l'hydroxyperoxyde de cyclohexyle, l'hydroperoxyde de cumène, l'hydroxyperoxyde d'éthylbenzène et l'hydroperoxyde de cyclohexylphényle.

8. Procédé suivant l'une quelconque des revendications 1 à 7, caractérisé en ce que le catalyseur au ruthénium et au chrome est présent sous la forme d'au moins un sel d'un acide carboxylique ou que le catalyseur comprend le mélange de nitrates ou de sulfates de ruthénium et de chrome.

9. Procédé suivant l'une quelconque des revendications 1 à 8, caractérisé en ce que la réaction de décomposition est effectuée à des températures comprises entre 25° et 250°C et à une pression compris'e entre 0,1 et 150 bars (0,1 et 150 atm.).

10. Procédé pour produire un mélange contenant des produits de décomposition d'un hydroperoxyde organique, dans lequel un hydrocarbure est oxydé en présence d'un gaz contenant de l'oxygène moléculaire pour fournir un mélange réactionnel contenant l'hydroperoxyde correspondant de cet hydrocarubre et cet hydroperoxyde est décomposé en présence de l'hydrocarbure de départ pour fournir un mélange contenant des produits réactionnels de cette réaction de décomposition comprenant des produits de type alcool et/ou cétone, caractérisé en ce que la réaction d'oxydation est conduite par mise en contact de l'hydrocarbure avec un gaz contenant de l'oxygène moléculaire en présence d'une quantité catalytique d'un système catalyseur comprenant du chrome et du ruthénium et que la réaction de décomposition est conduite par mise en contact d'un mélange réactionnel contenant l'hydroperoxyde de l'hydrocarbure de départ en présence d'un diluant avec une quantité catalytique de ce système catalyseur, éventuellement en présence d'un gaz contenant de l'oxygène moléculaire.

11. Procédé suivant la revendication 10, caractérisé en ce que le diluant est l'hydrocarbure soumis à l'oxydation dans l'étape d'oxydation de la réaction.

12. Procédé suivant la revendication 10 ou la revendication 11, caractérisé en ce que la réaction est effectuée à une température comprise entre 50° et 250°C.

13. Procédé pour l'oxydation d'un hydrocarbure pour produire un hydroperoxyde, caractérisé en ce qu'il comprend la mise en contact de cet hydrocarbure avec un gaz contenant de l'oxygène moléculaire à une température de 50° à 250°C en présence d'un quantité catalytique d'un système catalyseur comprenant du chrome et du ruthénium.

14. Procédé suivant l'une quelconque des revendications 10 à 13, caractérisé en ce que le système catalyseur présent dans l'étape d'oxydation représente une quantité de 0,01 ppm à 1000 ppm de ruthénium et de 0,01 à 1000 ppm de chrome.

15. Procédé suivant l'une quelconque des revendications 1 à 14, caractérisé en ce que le ruthénium et le chrome sont présents sous forme de composés solubles dans le mélange réactionnel.

16. Procédé suivant l'une quelconque des revendications 10 à 15, caractérisé en ce que le catalyseur au ruthénium et au chrome est présent sous la forme d'au moins un sel d'un acide carboxylique ou que le ruthénium et le chrome sont présents sous la forme de nitrate ou d'acétate.

17. Procédé suivant l'une quelconque des revendications 10 à 16, caractérisé en ce que l'hydrocarbure est choisi parmi l'isobutane, le cyclohexane, le cumène et l'éthylbenzène.